# EUROPEAN PATENT APPLICATION

(11) **EP 1 491 142 A1**
(43) Date of publication of application: **29.12.2004**
(21) Application number: 03745888.2
(22) Date of filing: 03.04.2003
(51) Int. Cl.: A61B 5/15

(54) **LANCET DEVICE**

(30) Priority: 04.04.2002 JP 2002102328
(71) Applicant: MATSUSHITA ELECTRIC INDUSTRIAL CO., LTD., Kadoma-shi, Osaka 571-8501 (JP)
(72) Inventor: WATANABE, Motokazu, Toyonaka-shi, Osaka 560-0025 (JP); YOSHIOKA, Toshihiko, Hirakata-shi, Osaka 573-0035 (JP)
(74) Representative: Grünecker, Kinkeldey, Stockmair & Schwanhäusser Anwaltssozietät
(86) International application number: PCT/JP2003/004251
(87) International publication number: WO 2003/084401

(57) **Abstract**

Provided is a lancet device capable of taking a sufficient amount of body fluid with less pain or readily healing wounds formed in the skin.

The lancet device is characterized by comprising a plurality of needles 2 for punctuating the skin, such as that of a finger tip of a diabetic patient. In the lancet device, the needles 2 are arranged at intervals of a predetermined distance or more, which prevents the wounds 20 formed by needle punctuation in the skin from connecting to each other.

## Description

### Technical Field

The present invention relates to a lancet device and a lancet for taking body fluid for diagnostic tests etc. from a skin surface, and further relates to a method of punctuating skin by using them.

### Background Art

Conventionally, lancet devices have been used as a tool for taking blood from skin surfaces of fingertips and the upper limbs for testing a blood sugar level and thelike. Generally, blood is collectedby a lancet device by slightly damaging a skin surface by a needle set in a device, followed by taking blood oozing from the injured site (see National Publication of International Patent Application No. 2000-509624, pages 5 to 7 and Figure 2). Note that the entire disclosure of the publication is incorporated herein by reference in its entirety.

However, when a large amount of blood ranging from 5 to 50 µl is required, the depth of the needle damaging the skin surface is controlled to increase the amount of blood in the conventional lancet device mentioned above. Because of this, the deeper a needle is inserted, the more pain the blood donor feels. Furthermore, since the needle is inserted into a single site, when a large amount of blood is taken, emerging blood cannot stay on the skin surface and slips out from the surface. In addition, it is hard to heal the wound resulting from the needle punctuation of the skin.

### Disclosure of the Invention

The present invention has been contrived in consideration of these circumstances. An object of the present invention is to provide a lancet device, lancet, and method of punctuating the skin, characterized in that the wound resulting from the needle punctuation of the skin is easily healed.

Another obj ect of the present invention is to provide a lancet derive, lancet, and method of punctuating the skin, characterized in that a sufficient amount of body fluid can be taken with less pain even when a large amount of body fluid is required.

To solve the above problem, a first invention of the present invention is a lancet device having a plurality of needles for punctuating the skin, wherein the plurality of needles are arranged at intervals of a predetermined distance or more.

A second invention of the present invention is the lancet device according to the first invention, wherein the predetermined distance is defined as one at which wounds formed in the skin by punctuation with the plurality of needles do not connect to each other.

A third invention of the present invention is the lancet device according to the first invention of the present invention, wherein the predetermined distance is defined as being 50% or more of the thickness of the plurality of needles.

A fourth invention of the present invention is the lancet device according to the first invention of the present invention, wherein the skin to be punctuated is one of a fingertip, the palm of the hand, an arm, and the belly.

A fifth invention of the present invention is the lancet device according to the first invention of the present invention, wherein the plurality of needles, each having a cut surface on the tip, are arranged such that the cut surfaces face the same direction,

A sixth invention of the present invention is the lancet device according to the first invention of the present invention, wherein the plurality of needles, each having a cut surface on the tip, are arranged such that the cut surfaces face outside.

A seventh invention of the present invention is the lancet device according to the first invention of the present invention, wherein the plurality of needles are hollow.

An eighth invention of the present invention is the lancet device according'to the first invention of the present invention, wherein the plurality of needles are solid.

A ninth invention of the present invention is the lancet device according to the first invention of the present invention, comprising
a lancet having a needle fixing portion for supporting the needles; and
a holder for holding the lancet
wherein the lancet is detachably provided to the holder.

A tenth invention of the present invention is the lancet device according to the ninth invention of the present invention, wherein the plurality of needles are arranged symmetric with respect to a point on the needle-fixing portion.

An eleventh invention of the present invention is the lancet device according to the ninth invention of the present invention, wherein the area of the opening portion provided in the holder for holding the lancet is 12 mm² or more.

A twelfth invention of the present invention is the lancet device according to the ninth invention of the present invention, wherein the opening portion is provided in a cap provided at the tip of the holder and the cap is configured to have the opening portion whose area can be changed.

A thirteenth invention of the present invention is a lancet device having a plurality of needles for punctuating the skin, in which the plane formed by connecting the tips of the needles is shaped so as to match the unevenness of the skin to be punctuated.

A fourteenth invention of the present invention is a lancet device according to the thirteenth invention, in which the length of each of the needles is adjusted so as to reach the epidermis but not to reach the dermis during punctuation.

A fifteenth invention of the present invention is a lancet having a plurality of needles for punctuating the skin, in which the needles are arranged at intervals of a predetermined distance or more, and the lancet can be detachably provided to the holder for holding the lancet.

A sixteenth invention of the present invention is a method of punctuating the skin by a plurality of needles arranged at intervals of a predetermined distance or more.

### Brief Description of the Drawings

Figure 1 is a perspective view of a lancet used in the lancet device according to Embodiment 1 of the present invention and a schematic illustration of a wound given by the lancet device;
Figure 2 is a sectional view showing the entire structure of the lancet device according to Embodiment 1 of the present invention;
Figure 3 is a perspective view of a lancet used in the lancet device according to Embodiment 2 of the present invention; and
Figure 4 is a sectional view of a gist portion of the lancet device according to Embodiment 2 of the present invention and a sectional view of the skin surface punctuated with the lancet device.

### Explanation of reference numerals

- 2: Needle
- 3: Needle-fixing portion
- 4: Holder
- 5: Cap
- 6: Holder guide
- 7, 11: Spring
- 8: Switch
- 9: Stopper
- 10: Step portion
- 12: Plunger
- 13: Opening portion
- 14: Horny layer
- 15: Epidermis
- 16: Dermis
- 17: Skin surface layer
- 18: Capillary vessel
- 20: Wound
- 21: Cut surface
- 22, 24: Lancet
- 23, 25: Lancet device

### Best Mode for Carrying Out the Invention

Now, the embodiments of the present invention will be explained.

### (Embodiment 1)

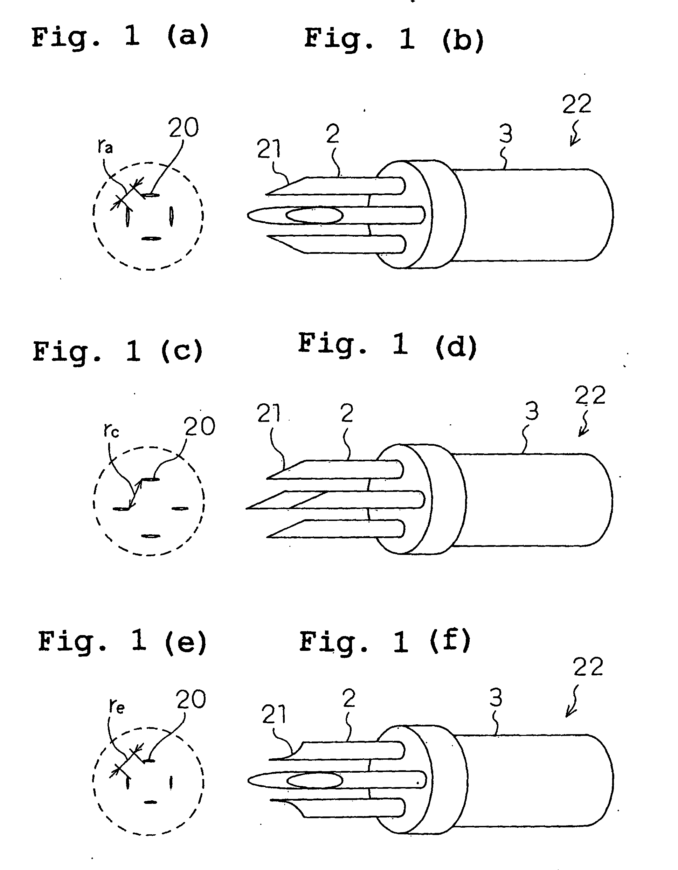

Figures 1(b), 1(d) and 1(f) are perspective views of the structures of lancets of the present invention each having needles and a needle holding portion. Referring to these figures, the structure and operation of the lancet of the present invention will be explained below.

Figure 1(b) shows a lancet having four needles 2, each having a blade-form cut surface 21 on its tip, fixed on a needle-fixing portion 3. More precisely, the needles 2 are fixed on the needle-fixing portion 3 such that the cut surfaces 21 face distally from the center axis of the needle-fixing portion 3 (in other words, the surface faces outward). The blade-form used herein refers to a cut surface of the needle tip 2 having a flat surface. Since the needles are arranged such that the cut surfaces look outside, the tip forms of the needles are more easily checked after the lancet is manufactured. This is advantageous in consideration of quality control. In Figure 1(d), four needles 2, each having a blade-form tip, are fixed on the needle-fixing portion 3 such that the cut surfaces 21 face nearly the same direction. In Figure 1 (f) , four needles 2, each having a cut surface 21 with a tuck-form tip, are fixed on the needle-fixing portion 3 such that the cut surfaces 21 face distally from the center axis of the needle-fixing portion 3. The tuck-form of the needle 2 used herein refers to a cut surface of the needle tip not having a flat plane but a depressed one.

The blade-form has the advantage that it is easily manufactured; whereas the advantage of the tuck-form is that the shape of the resulting wound 20 becomes sharper than ever, making it easier to heal the wound.

Figures 1(a), 1(c), and 1(e) are schematic views of wounds 20 formed by punctuation with lancets 22 shown in Figures 1(b), 1(d), and 1(f), respectively. When the tip of the needle 2 is blade-form or tuck-form, a long-and-thin wound parallel to the blade or tuck surface is resulted. On the other hand, when the tip of the needle 2 is conical, the wound is not long-and-thin but takes an irregular shape. Because of the irregular shape, a long time is required to close up or heal the wound. Furthermore, if a plurality of wounds 20 formed by needles 2 are mutually connected to develop into a larger wound 20, it takes time to heal the wound. Then, in the present invention, the orientations of wounds 20 are adjusted so as not to cross each other', as shown in Figure 1(a) or 1(e), or arranged in parallel to each other, as shown in FIG. 1(c), thereby preventing mutual connection of the wounds 20.

In either case, a plurality of needles 2 are arranged on the needle-fixingportion 3 at a predetermined interval or more in order to prevent the wounds 20 formed in the skin from connecting to each other. The predetermined interval is indicated by reference symbol ra in Figure 1(a), rc in Figure(c), and re in Figure 1(e). Wounds are desirably formed, in the skin of any part of the body, at predetermined intervals that prevent the wounds from connecting to each other. For example, when a fingertip of a diabetic patient is punctuated, the interval is desirably 50% or more of the needle 2 thickness. As the thickness of a needle, 20 to 40 gage may be used. Any material may be used as long as it has the strength sufficient to punctuate the skin. The needles used in conventional lancet devices may be used without any particular limitation.

Particularly, the lancet 22 shown in Figure 1(c) is preferable since it is likely to form wounds 20 discretely from each other.

As described above, when the lancet 22 is used, even if a plurality of wounds 20 are formed by punctuation with needles 2, they are rarely connected with each other to develop into a large wound. Therefore, the lancet 22 has the advantage in that the wounds are easily healed. Furthermore, since the wounds 20 do not grow large, pain can be reduced.

In either case, not less than two needles 2 are fixed on a single needle-fixing portion 3 while being arranged in parallel lengthwise, and the needle tips are positioned in the same imaginary plane formed in perpendicular to the length direction of the needles. For the reason, when the surface of the skin is flat, the skin is punctuated simultaneously by the needles 2 and the patient feels pain only once.

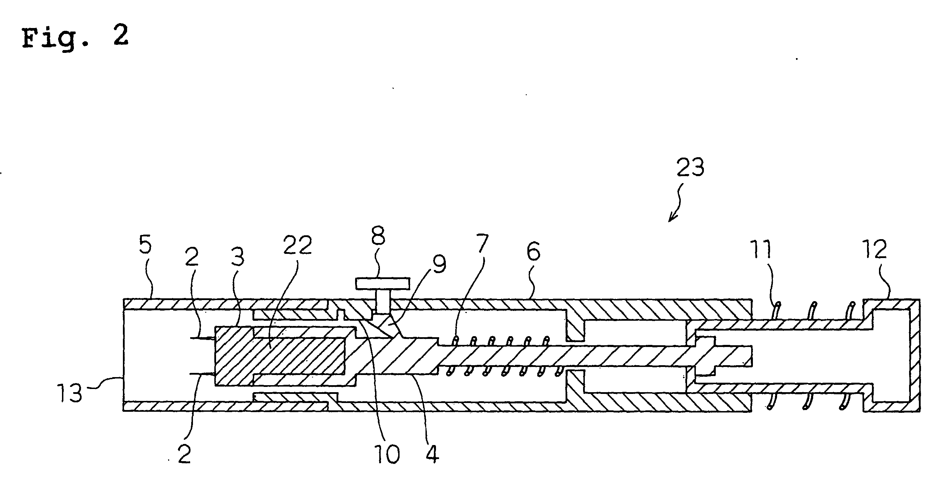

Figure 2 is a sectional view showing the entire structure of a lancet device 23 installing a lancet 22 shown in Figure 1. The needles 2 are arranged at regular intervals and fixed on the needle-fixing portion 3, which is detachably immobilized on a holder 4. The needle-fixing portion 3 integrated with the needle 2'can be removed and discarded after blood collection. Thus, the operationality of the lancet device is good. Furthermore, various kind of needle-fixing portion 3 with different number of needles 2, or with different intervals among the needles 2 can be attached to the holder 4. Since the extension force of a spring is used for punctuation, a patient feels a moment's pain.

The elastic spring 7 links the holder 4 and a holder guide 6 and plays a role in ejecting the needles 2 toward an opening portion 13. A cap 5 can be removed from the holder guide 6, so that the needles 2 and the needle-fixing portion 3 can be attached or removed. The cap 5 plays a role in guiding the needle 2 toward the opening portion 13 in the same manner as the holder guide 6.

To immobilize the spring at a compressed state, a stopper 9 attached to the holder 4 is usedas immobilization means. When the stopper 9 is hooked on a step portion 10 formed on the holder guide 6, the spring 7 is immobilized at the compressed state. A plunger 12 is connected to the holder guide 6 via a spring 11. The spring 11 is immobilized at a compressed state by moving the holder 4.

Blood is collected by bringing the opening portion 13 of the cap 5 in touch with the skin. Punctuation is performed as follows. When a switch 8 presses the stopper 9, immobilization of the stopper 9 at the step portion 10 is released. As a result, the needles 2 are ejected toward the opening portion 13 by the elastic force of the spring 7.

Now, how to use the lancet device 23 shown in Figure 2 will be explained. First, the plunger 12 is pulled to immobilize the stopper 9 onto the step portion 10. In this state, when the switch 8 is pressed, the immobilization of the stopper 9 is released. As a result, the holder 4 is ejected toward the opening portion 13 by the elastic force of the spring 7. At this time, if the opening portion 13 is pressed against the skin surface of an upper arm, a finger, or the like, the needle 2 damages the skin surface. In this manner, blood can be taken. Since the extension force of the spring is used for punctuation, the patient feels moment's pain at a time.

### (Example 1)

Blood was collected from an upper arm and a finger by using the lancet device of this embodiment in accordance with the aforementioned method. In this example, the needle 2 and the needle-fixing portion 3 shown in Figure 2 (b) were used. The thickness of the needle was set at 28 gage and the blood amount at 50 µl. As a comparative example, 50 µl of blood was taken in the same manner by using a conventional lancet having a single needle of 28 gage. Even if the depth of the needle 2 inserted into the skin is reduced in the lancet of this example, compared to the comparative example, the same amount of blood was taken with less pain during punctuation.

### (Embodiment 2)

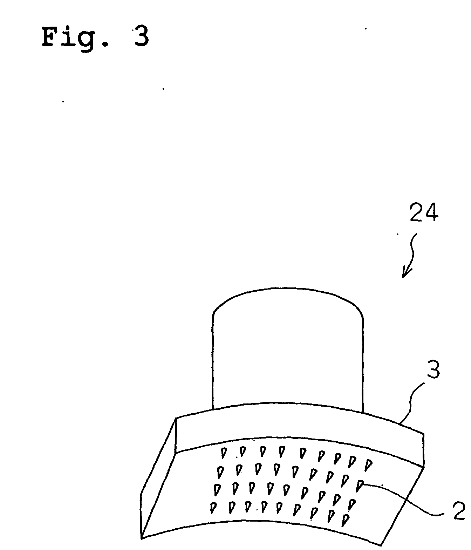

Figure 3 shows a lancet to be installed in the lancet device according to Embodiment 2 of the present invention. The lancet 24 of this embodiment is used for taking an interstitial fluid. The lancet 24, which has numeral needles 2 and a needle-holding portion 3, is installed, for example, in the holder 4 of the lancet shown in Figure 2 when used. Needles 2 have the same length. However, since the surface of the needle-holding portion 3 for holding the needles 2 is curved, the tips of the needles 2 are naturally positioned on an imaginary curved surface.

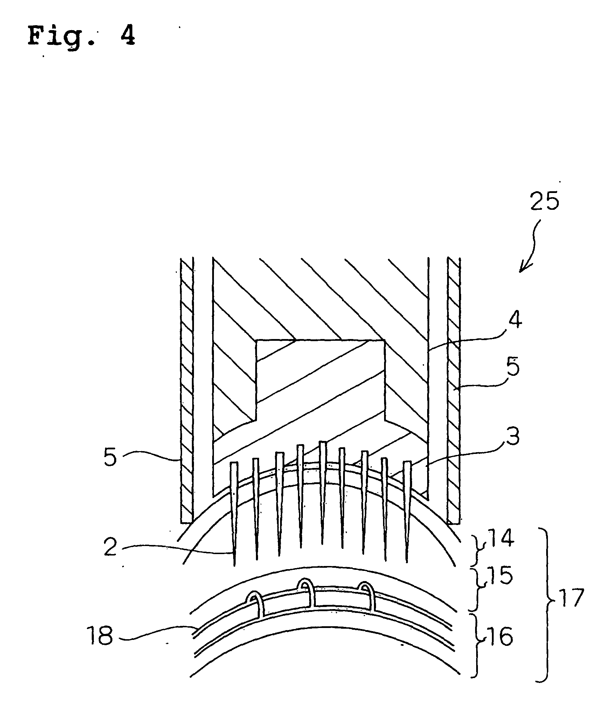

Figure 4 is a schematic sectional view of the gist portion of a lancet device 25 having the lancet 24 shown in Figure 3 and illustrates the state of the skin surface punctuated by the lancet device 25. A skin surface layer 17 is composed of the outermost horny layer 14, the epidermis 15 having no capillary vessel, and dermis 16 having a capillary vessel. The thickness of the needle 2 is preferably smaller than 28 gage. Since pain is reduced, the thinner the thickness of the needle 2, the more preferable. However, when the needle 2 is too thin, the strength becomes low. Therefore, the ratio of length to width of the needle 2 is preferably from 10:1 to 2:1, where the width is measured at the position of the needle 2 in contact with the needle-holding portion 3.

The interstitial fluid is taken from the curved surface of the patient's finger, arm, belly and so forth. When the lancet device having a sufficiently large area is used, and the tips of the needles 2 are placed on the same imaginary flat plane, some needles 2 are inserted deeper, whereas other needles 2 are inserted but insufficiently. On the other hand, according to the present invention as shown in Fig. 4, the imaginary surface connecting the needle 2 tips is formed to match with the uneven skin surface to be punctuated. Therefore, even if the skin surface is curved, all needles 2 reach the epidermis, enabling collection of the interstitial fluid efficiently. In addition, it is not probable that, for example, only the needles 2 of the center portion are inserted deeply into the skin during punctuation. Therefore, the pain is low. Since the capillary vessel 18 present in the dermis is not damaged, the interstitial fluid can be efficiently taken without taking blood.

According to the lancet device according to this embodiment, the interstitial fluid is taken from a plurality of wounds 20. Therefore, a relatively large amount of interstitial fluid can be taken compared to the case of taking it from a single wound. In addition, since the needles 2 are very short, the pain is minimized.

Incidentally, the needle 2 of a solid body was used to reduce thickness; however, a hollow needle 2 may be used. Furthermore, the interstitial fluid may be taken by punctuating the skin surface by a solid or hollow needle 2, followed by sucking the skin surface by suction means or pressing the punctuation portion by fingers or the like.

### (Example 2)

A non-hollow (solid) needle was selected as the needle 2 and the length was set at 0.15 mm so as not to reach the dermis 16 of the skin surface layer 17. The needle 2 having a thickness of 33 gage was used. The imaginary curved surface formed with the tips of the needles 2 was shaped so as to match with that of a patient's fingertip. The patient's fingertip was punctuated by the lancet device thus formed. As a comparative example, needles 2 whose tips are arranged on an imaginary flat plane were used. As a result, the lancet device having needles 2 whose tips form an imaginary curved plane gave less pain than that having the needles 2 whose tips form an imaginary flat plane.

In the lancet devices and lancets employed in the embodiments so far described, the number of needles 2 is not particularly limited as long as it is present at least two.

Furthermore, the cut surface 21 may not be formed in all needles 2. In other words, the cut surface 21 may be formed in some of the needles. Any shaped cut surface 21 other than a blade-form or tuck form may be employed. Furthermore, no cut surface 21 may be formed. In this case, as long as a plurality of needles 2 are arranged at predetermined intervals or more, the same effects as those of the lancet device according to embodiment 1 can be obtained.

In Embodiment 1, the imaginary surface formed by connecting the tips of the needles 2 of the lancet device is defined as a plane; however, it may have a shape that matches with the uneven surface of the skin to be punctuated as in the case of Embodiment 2. In that case, even if a fingertip is punctuated, pain can be further minimized.

Embodiment 2 shows a case of taking the interstitial fluid. However, the lancet device according to Embodiment 2 may be configured to take blood. In this case, it is sufficient that the length of the needle 2 may reach the capillary vessel 18 from the skin surface to be punctuated. In this case, the same effects as mentioned above can be obtained with less pain.

In the lancet device of Embodiment 2, if the needles 2 are arranged at predetermined intervals or more, the resulting wounds 20 formed in the skin can be readily healed similarly to Embodiment 1.

In the lancet 24 to be used in the lancet device of Embodiment 2, the lengths of the needles 2 may be arranged so as to match with the unevenness of the skin in place of being arranged on the curved surface of the needle-holding portion 3 with the same length.

In the lancet devices explained in,the foregoing, if the needles 2 are arranged symmetric with respect to a point on the needles holding portion 3, the entire lancet device can be miniaturized.

According to the lancet device of Embodiment 1 or 2, since at least two needles 2 are used, a sufficient amount of body fluid can be taken even if the needles are inserted into the skin shallower than a conventional case using a single needle. As a result, the pain during the punctuation can be reduced.

Since the body fluid is spread over a broader area of the skin surface compared to the case of taking it from a single site, the lancet device of the present invention has the advantage that the body fluid thus taken rarely slips down from the skin surface.

In the lancet device according to Embodiment 1, the needle 2 may be hollow or solid. In the case of the hollow needle 2, the body fluid may be sucked up through the interior of the needle. Whereas, in the case of the solid needle 2, since it is not necessary to form a hollow portion inside, the thickness of the needle may be reduced. When the thinner needle 2 is used, pain can be lessened.

In the lancet devices of Embodiments 1 and 2, the opening portion 13 is provided in the cap 5 formed at the tip of the holder 4. The cap 5 may be constructed to have the opening portion 13, the area of which may be changed.

In this case, the area of the opening portion 13 is preferably 12 mm² or more. If the area of the opening portion is set as defined above, it is possible to prevent the body fluid from depositing on the lancet device, when a relatively high viscosity body fluid oozing out of the skin surface spreads over the surface in taking a large amount of body fluid (5 to 50 µl).

As the body fluid to be taken by the lancet device of the present invention, a fluid such as blood plasma and lymph fluid may be included in addition to blood and interstitial fluid.

In the lancet devices of the present invention explained in the foregoing, a relatively large amount of body fluid ranging from 5 to 50 µl was taken. However, even when a small amount of body fluid, e.g., 5 µl or less, is collected, the same effects as above can be obtained.

## Claims

1. A lancet device having a plurality of needles for punctuating the skin, wherein the plurality of needles are arranged at intervals of a predetermined distance or more.

2. The lancet device according to claim 1, wherein the predetermined distance is defined as one at which wounds formed in the skin by punctuation with the plurality of needles do not connect to each other.

3. The lancet device according to claim 1, wherein the predetermined distance is defined as being 50% or more of the thickness of the plurality of needles.

4. The lancet device according to claim 1, wherein the skin to be punctuated is one of a fingertip, the palm of the hand, an arm, and the belly.

5. The lancet device according to claim 1, wherein the plurality of needles, each having a cut surface on the tip, are arranged such that the cut surfaces face the same direction,

6. The lancet device according to claim 1, wherein the plurality of needles, each having a cut surface on the tip, are arranged such that the cut surfaces face outside.

7. The lancet device according to claim 1, wherein the plurality of needles are hollow.

8. The lancet device according to claim 1, wherein the plurality of needles are solid.

9. The lancet device according to claim 1, comprising
a lancet having a needle fixing portion for supporting the needles; and
a holder for holding the lancet
wherein the lancet is detachably provided to the holder.

10. The lancet device according to claim 9, wherein the plurality of needles are arranged symmetric with respect to a point on the needle-fixing portion.

11. The lancet device according to claim 9, wherein the area of the opening portion provided in the holder for holding the lancet is 12 mm² or more.

12. The lancet device according to claim 9, wherein the opening portion is provided in a cap provided at the tip of the holder and the cap is configured to have the opening portion whose area can be changed.

## Amended claims

### Amended claims under Art. 19.1 PCT

1. (AMENDED) A lancet device having a plurality of needles for punctuating the skin, in which the plane formed by connecting the tips of the needles is shaped so as to match the unevenness of the skin to be punctuated.

2. The lancet device according to claim 1, wherein the predetermined distance is defined as one at which wounds formed in the skin by punctuation with the plurality of needles do not connect to each other.

3. The lancet device according to claim 1, wherein the predetermined distance is defined as being 50% or more of the thickness of the plurality of needles.

4. The lancet device according to claim 1, wherein the skin to be punctuated is one of a fingertip, the palm of the hand, an arm, and the belly.

5. (Amended) A lancet device according to claim 1, in which the length of each of the needles is adjusted so as to reach the epidermis but not to reach the dermis during punctuation.

6. The lancet device according to claim 1, wherein the plurality of needles, each having a cut surface on the tip, are arranged such that the cut surfaces look outside.

7. The lancet device according to claim 1, wherein the plurality of needles are hollow.

8. The lancet device according to claim 1, wherein the plurality of needles are solid.

9. The lancet device according to claim 1, comprising
a lancet having a needle fixing portion for supporting the plurality of needles; and
a holder for holding the lancet
wherein the lancet is detachably provided to the holder.

10. (DELETED)

11. The lancet device according to claim 9, wherein the area of the opening portion provided in the holder for holding the lancet is 12 mm² or more.

12. The lancet device according to claim 9, wherein the opening portion is provided in a cap provided at the tip of the holder and the cap is configured to have the opening portion whose area can be changed.
